# EUROPÄISCHE PATENTSCHRIFT

(11) **EP 1 108 996 B1**
(45) Veröffentlichungstag und Bekanntmachung des Hinweises auf die Patenterteilung: **27.09.2006**
(21) Anmeldenummer: 00126456.3
(22) Anmeldetag: 07.12.2000
(51) Int. Cl.: G01J 3/46, G01N 33/32, G01N 21/01

(54) **Verfahren zur Messung optischer Parameter an flüssigen Medien**
Method for measuring optical parameters of liquid samples
Procédé pour mesurer des paramètres optiques de substances liquides

(30) Priorität: 17.12.1999 DE 19960919
(43) Veröffentlichungstag der Anmeldung: 20.06.2001
(73) Patentinhaber: E.I. DU PONT DE NEMOURS AND COMPANY, Wilmington, Delaware 19898 (US)
(72) Erfinder: Hustert, Hans-Hendrik, 44227 Dortmund (DE)
(74) Vertreter: Gille Hrabal Struck Neidlein Prop Roos

(56) Entgegenhaltungen:
- WO-A-97/21998
- DE-A- 3 029 257
- DE-A- 4 100 789
- US-A- 5 933 685
- PATENT ABSTRACTS OF JAPAN vol. 1996, no. 09, 30. September 1996 (1996-09-30) & JP 08 128898 A (TOPPAN PRINTING CO LTD), 21. Mai 1996 (1996-05-21)

## Beschreibung

Die Erfindung betrifft ein Verfahren zur Messung optischer, insbesondere farbmetrischer Parameter an flüssigen, farbigen Medien, insbesondere an Naßlacken.

In der Lackindustrie ist es in den verschiedenen Produktions-, Standardisierungs- und Entwicklungsstufen der Lackherstellung und Lackentwicklung zwecks Qualitätskontrolle erforderlich, daß an den Lacken optische Messungen durchgeführt werden. Man erhält so Aussagen darüber, ob die Lacke den geforderten, optischen Parametern, z.B. der geforderten Farbstärke, entsprechen und kann erforderliche Korrekturschritte anschließen. Die Messung kann dabei prinzipiell an Naßlacken oder an applizierten und getrockneten bzw. gehärteten Lacken erfolgen. In der Lackstandardisierung, z.B. bei der Herstellung von standardisierten Autoreparatur-Mischlacken oder -Pigmentpasten ist die Messung an applizierten und gehärteten Lacken, z.B. an beschichteten Blechen, unumgänglich, da diese Methode im Vergleich zur Messung an Naßlacken das Erscheinungsbild des durch Trocknung oder Härtung fertiggestellten Lackfilmes erfaßt und es letztlich auf den Farbeindruck des applizierten Lackes ankommt und nicht auf den des Naßlackes.

Die Messung an applizierten Lacken ist allerdings relativ aufwendig, da im Vorfeld der Messung zunächst beschichtete Bleche, sogenannte Prüf- oder Mustertafeln erstellt werden müssen. Dazu muß beispielsweise bei Unifarbtönen zunächst von jedem Farbton eine Weißlackabmischung hergestellt werden, die dann appliziert und getrocknet bzw. gehärtet wird. Bei der Standardisierung von Pigmentpasten kommt als zusätzlicher Arbeitsschritt noch das Auflacken mit entsprechenden Bindemitteln vor der Applikation hinzu.

Bei bestimmten Anwendungen, wie z.B. bei ersten Prüfschritten in der Standardisierung oder Lackfertigung sowie zur Abfüllkontrolle, ist es jedoch ausreichend, zur Ermittlung von Zwischenergebnissen die farbmetrischen Messungen zunächst am Naßlack vorzunehmen, was wesentlich schneller und kostengünstiger erfolgen kann als Messungen am applizierten gehärteten Lack. Einsetzbare Farbmeßgeräte sind bekannt und im Handel erhältlich. Ebenso sind entsprechende Meßzellen zur Naßlackmessung bekannt. Es handelt sich hierbei beispielsweise um zylinder- oder quaderförmige Meßküvetten aus Glas, die vor der Meßöffnung des Farbmeßgerätes plaziert werden. Nachteil dieser einfachen Meßzellen ist, daß die Genauigkeit der mit ihnen erhaltenen Meßergebnisse unbefriedigend ist, beispielsweise hervorgerufen durch rasches Absetzen des Lackes in der Küvette oder durch den Einfluß des Zwischenmediums Glas, beispielsweise auf Grund physikalischer Wechselwirkungen.

Desweiteren wird in der DE-A-2531459 ein Verfahren zur Farbmessung an flüssigen Lacken beschrieben, wobei der Lack mittels eines Überlaufgefäßes, welches anhebbar in einem Mischbehälter mit Lack angeordnet ist, aus dem Mischbehälter entnommen und in den Lack im Überlaufgefäß frischer Lack aus dem Mischbehälter über eine Leitung eingebracht und die Oberfläche des im Überlaufgefäß befindlichen Lackes farbmetrisch gemessen wird.

In der DE-A-2525701 wird ein weiteres Verfahren zur Farbmessung an flüssigen Lacken beschrieben. In diesem Verfahren wird ein kontinuierlicher dünner Film aus dem zu untersuchenden Lack gebildet und ein Abschnitt dieses Films farbmetrisch gemessen. Der Lackfilm kann dabei ein mit einer Unterlage wandernder Film oder ein über eine Unterlage mit laminarer Strömung hinweg wandernder Film sein. Im ersten Fall handelt es sich bei der Unterlage um eine um eine horizontale Achse drehbare Meßscheibe, im zweiten Fall um einen plattenförmigen Körper mit etwa senkrecht stehender Oberfläche. In beiden Fällen wird der Lack mittels eines Gießkastens mit Austrittspalt auf die jeweilige Unterlage aufgebracht. Nachteilig an diesem Verfahren ist im Falle der Verwendung einer drehbaren Meßscheibe, daß mit einer akzeptablen Scheibengröße nur dann gearbeitet werden kann, wenn Farbmeßgeräte eingesetzt werden, die eine mehr seitlich am Gehäuse angeordnete Meßöffnung aufweisen. Bei Farbmeßgeräten mit mittig angeordneten Meßöffnungen müssen extrem große Scheiben eingesetzt werden. Im Falle der Verwendung des senkrecht stehenden plattenförmigen Körpers kann es aufgrund der fehlenden Scherung zu Flokkulationseffekten kommen. Ebenso müssen der Einfluß der Schwerkraft und die damit verbundenen Nachteile in Kauf genommen werden. So kann beispielsweise nur mit Naßlackschichtdicken oberhalb der Ablaufgrenze gearbeitet werden.

Ein weiteres Verfahren ist aus dem Dokument DE 30 29 257 A1 bekannt. Hier wird zur kontinuierlichen Kontrolle der Farbwerte eines farbigen Anstrichmittels, ein Teil der Farbe auf eine Walzen/Band-Kombination aufgetragen und mit einer Messeinrichtung werden optische Parameter, gemessen.

Auf dem Gebiet der Farbkopierer, siehe US 5 933 685, ist es ferner bekannt ein Tonerfilm auf einen walzenförmigen Träger aufzubringen und die Konzentration mit einem Detektor zu messen.

Aufgabe der Erfindung war es daher, ein Verfahren zur Messung optischer, insbesondere farbmetrischer Parameter an flüssigen, farbigen Medien, insbesondere an Naßlacken, bereitzustellen, welches eine schnelle und effektive Messung der optischen Parameter ermöglicht und welches gleichzeitig eine beispielsweise zur Qualitätskontrolle erforderliche Meßgenauigkeit gewährleistet. Das Verfahren soll so gestaltet sein, daß Meßgeräte mit beliebiger Anordnung der Meßöffnung optimal einsetzbar sind.

Die Aufgabe wird gelöst durch ein Verfahren nach Anspruch 1, zur Messung optischer, insbesondere farbmetrischer Parameter an flüssigen, farbigen Medien, insbesondere an Naßlacken, welches dadurch gekennzeichnet ist, daß das flüssige, farbige Medium mittels einer Küvette auf einen sich in kontinuierlicher Bewegung befindlichen, walzenförmigen Träger aufgegossen, ein Film aus dem flüssigen, farbigen Medium auf dem sich in kontinuierlicher Bewegung befindlichen walzenförmigen Träger ausgebildet und an dem Film aus dem flüssigen, farbigen Medium optische, insbesondere farbmetische Parameter gemessen werden. Dabei wird der Auftrag durch eine spaltförmige Öffnung, wie im Anspruch 1 definiert, aufgetragen.

Als zu messende optische Parameter kommen beispielsweise Helligkeit und farbmetrische Parameter, wie Farbstärke oder Farbabstand in Frage.

Bei den flüssigen, farbigen Medien handelt es sich insbesondere um flüssige Lacke. Im folgenden soll daher zur Vereinfachung immer der Begriff Lack verwendet werden, wobei jedoch in diesen Begriff grundsätzlich auch andere flüssige Medien einbezogen sein sollen.

Das Aufbringen des Lackes auf den walzenförmigen (zylinderförmigen) Träger, im folgenden kurz Walze genannt, erfolgt mittels Aufgießen mittels einer Küvette.

Nach dem Aufbringen des Lackes auf die Walze kann die Messung erfolgen. Um das Meßgerät vor Lackverunreinigungen zu schützen, wird die Walze bevorzugt erst kurz vor der Messung direkt vor der Meßöffnung eines entsprechenden Meßgerätes positioniert. Ebenso ist es aber möglich Walze und Meßgerät in dem zur Messung erforderlichen Abstand miteinander zu verbinden und in dieser Position den Lack aufzubringen.

Im folgenden soll das erfindungsgemäße Verfahren näher beschrieben werden.

In einem ersten Verfahrensschritt wird der Lack in geeigneter Weise auf die sich bereits in rotierender Bewegung befindliche Walze aufgebracht. Die Walze ist bevorzugt horizontal oder vertikal bezüglich der Meßöffnung des Meßgerätes angeordnet. Die horizontale Anordnung kann z.B. bei sehr hochviskosen Lackmaterialien eingesetzt werden. Im Allgemeinen ist jedoch die vertikale Anordnung bevorzugt. Die Walze wird mit einem von einer Antriebsvorrichtung antreibbaren Getriebe in rotierende Bewegung versetzt.

Die im erfindungsgemäßen Verfahren als Träger für den Lack einzusetzende Walze kann in verschiedenen Ausführungsformen vorliegen. Zum Beispiel kann es sich bei der Walze um einen Hohlzylinder mit variabler Wandstärke oder einen Vollkörperzylinder, wobei ein Vollkörperzylinder aus Gewichtsgründen im Inneren ausgespart sein kann, handeln. Die Walze besteht bevorzugt aus Metall, besonders bevorzugt aus Edelstahl. Die Abmessungen der Walze können innerhalb gewisser Grenzen variabel gestaltet werden. Die Breite des Walzenzylinders richtet sich beispielsweise nach Art und Größe von Meßgerät, insbesondere Farbmeßgerät und Meßöffnung. Die Walze mit dem aufgebrachten Lackfilm wird vor der Meßöffnung des entsprechenden Meßgerätes positioniert. Der Lackfilm soll in seiner Breite die Meßöffnung und bevorzugt auch die Umgebung der Meßöffnung abdecken. Die Breite des Lackfilms wird dabei wiederum beispielsweise von Größe und Aufbau der Vorrichtung, mit der der Lack auf die Walze gebracht werden kann, bestimmt. Auf die Ausgestaltung dieser bevorzugt einzusetzenden Vorrichtung wird nachfolgend noch detailliert eingegangen. Die Breite der Walze soll solche Maße aufweisen, daß beidseitig neben dem auf der Walze ausgebildeten Lackfilm lackfilmfreie Bereiche der Walze, verbleiben. Beispielsweise sollen auf jeder Seite etwa 5 bis 30 %, bezogen auf die Breite des Naßlackfilms, an freier Walzenfläche verbleiben. Der Durchmesser der Walze soll bevorzugt so gewählt werden, daß die Krümmung des Zylinders bezogen auf die Größe des Meßfleckes vernachlässigt werden kann, so daß man von einer ebenen Fläche innerhalb des Meßfleckes ausgehen kann.

Die Größe des vom Meßgerät erfaßten Meßfleckes kann bei verschiedenen Meßgeräten, insbesondere bei Farbmeßgeräten unterschiedlich sein und hängt beispielsweise davon ab, ob unifarbene Lacke (mit Absorptionspigmenten pigmentierte Lacke) oder Effektlacke (mit plättchenförmigen Metalleffektpigmenten pigmentierte Lacke) gemessen werden sollen. Beispielsweise kann man bei der Messung von unifarbenen Lacken von einer ebenen Fläche innerhalb des Meßfleckes ausgehen bei einem Walzendurchmesser von beispielsweise größer/gleich 15 cm, bevorzugt größer/gleich 20 cm. Nach oben ist der Walzendurchmesser beispielsweise dadurch begrenzt, daß, auf den aufgebrachten Naßlackfilm bezogen, nicht zu große Verdunstungsflächen entstehen sollen. Vorzugsweise kann der Walzendurchmesser 18 bis 30 cm betragen.

Das Aufbringen des Lackes auf die rotierende Walze erfolgt wie vorstehend bereits genannt, mit Hilfe eines Behälters in Form einer Küvette. Der Behälter kann an jeder beliebigen Stelle des Walzenumfanges angeordnet sein. Er kann sich z.B. auf der gleichen Seite der Walze wie das Meßgerät befinden, d.h. in unmittelbarer Nähe des Meßgerätes, er kann sich jedoch auch weiter entfernt vom Meßgerät befinden, z.B. auf der entgegengesetzten Seite der Walze. Bevorzugt befindet sich der Behälter auf der entgegengesetzten Seite der Walze, so daß sich Meßgerät und Behälter im wesentlichen in einem Abstand voneinander befinden, der in etwa dem Walzendurchmesser entspricht. Durch letzgenannte Anordnung können eventuell durch den Lackauftrag hervorgerufene Verschmutzungen des Farbmeßgerätes und speziell der Meßöffnung vermieden werden.

Ein bevorzugt geeigneter Behälter zum Aufbringen des Lackes weist eine Einfüllöffnung für den Lack und eine Austrittsöffnung auf, über die der Lack auf die Walze gelangen kann. Dabei handelt es sich bei der Austrittsöffnung um eine spaltförmige Austrittsöffnung. Bei dem Behälter handelt es sich um eine Küvette, die vorzugsweise etwa würfel- oder quaderförmig ausgebildet ist. Die Küvette kann oben offen oder geschlossen sein. Es muß sich jedoch in der Küvette eine geeignete Einfüllöffnung zum Einfüllen des Lackmaterials befinden. Bevorzugt wird daher eine würfel- oder quaderförmige an der Deckseite offene Küvette mit einer spaltförmigen Austrittsöffnung verwendet.

Im folgenden soll bei der Beschreibung des erfindungsgemäßen Verfahrens insbesondere auf die bevorzugte Ausführungsform mit einer vertikal vor der Meßöffnung des Farbmeßgerätes angeordneten Walze und mit einer Küvette als Auftragsvorrichtung Bezug genommen werden.

Damit der Lack auf die Walze gelangen kann, wird die Küvette zunächst dicht an die Walze herangeführt. Um die Küvette paßgenau an die Walze heranführen und mit ihr in Berührung bringen zu können, weist die Küvette bevorzugt auf einer ihrer vertikalen Begrenzungsflächen eine der Walzenkrümmung angepaßte Krümmung auf. Die spaltförmige Öffnung, über die das Lackmaterial aus der Küvette auf die Walze gelangen kann, befindet sich vorzugsweise an der Kante zwischen der Bodenfläche und der gekrümmten Küvettenbegrenzungsfläche. Die spaltförmige Öffnung erstreckt sich dabei über die gesamte Innenbreite der Küvette. Auf die Küvettenwände erstreckt sich die spaltförmige Öffnung jedoch nicht. Die spaltförmige Öffnung weist in ihrer gesamten Längsausdehnung die gleiche Spaltbreite auf. Bevorzugt ist die Küvette so ausgestaltet, daß sie an ihrer der Walze zugewandten Seite vollständig offen ist. In diesem Fall weisen dann die der Walze zugewandten Seitenwände an ihrer Schmalseite eine der Walzenkrümmung angepasste Krümmung auf und liegen an der Walze an.

Die spaltförmige Öffnung in der Küvette kann beispielsweise realisiert werden, indem der Boden der Küvette einen geeigneten Abstand von der Walze aufweist. In diesem bevorzugten Fall einer auf ihrer der Walze zugewandten Seite offenen Küvette wird der Austrittsspalt durch die Walze und den Boden der Küvette begrenzt.

Im vorstehend genannten Fall wird die Küvette so an die Walze herangeführt und mit ihr in Berührung gebracht, daß die Seitenwände mit ihrer gekrümmten Schmalseite an der Walze bündig anliegen. Dabei kann die Küvette vorzugsweise mittels einer Haltevorrichung und/oder durch geeignete Stellelemente, z.B. Federelemente, in der gewünschten Position gehalten werden.

Da das Lackmaterial über die spaltförmige Öffnung im Bodenbereich der Küvette auf die Walze gelangt, entspricht die Spaltlänge der Breite des auf der Walze ausgebildeten Naßlackfilms und die Spaltbreite entspricht der aufgebrachten Naßfilmschichtdicke. Die Spaltbreite wird so bemessen, daß die erforderliche Schichtdicke daraus resultiert.

Aus dem vorstehend gesagten ergibt sich, daß Walzen- und Küvettengröße vorteilhafterweise aneinander angepaßt sind. Die Breite der Küvette soll vorzugsweise etwa 40 bis 90 %, besonders bevorzugt 50 bis 80 % der Breite des Walzenzylinders betragen.

Die Küvette kann aus verschiedenen Materialien, z.B. aus Kunststoff oder aus Metall gefertigt sein. Als Kunststoffe kommen dabei z.B. Polypropylen, Polyethylen, Polycarbonat, Polyethylenterephthalat oder Polyamid einzeln oder in Mischung in Frage. Als Metalle kommen z.B. Aluminium, Kupfer, Zink oder auch Metalllegierungen wie Kupfer- oder Aluminiumlegierungen, z.B. Messing in Frage. Bevorzugt besteht die Küvette jedoch aus Kunststoff. Besonders bevorzugt kann der Boden der Kunststoffküvette aus Metall oder einer Metalllegierung bestehen, beispielsweise den vorstehend genannten. Bevorzugt kann der Boden aus Kupfer oder Messing sein. Bei Einsatz eines Bodens aus Metall oder einer Metalllegierung ist eine besonders exakte Kanteneinstellung der spaltförmigen Öffnung der Küvette und damit der Schichtdicke des Naßlackfilmes möglich.

Die Walze wird mit Hilfe eines Motors in rotierende Bewegung versetzt. Die Drehgeschwindigkeit der Walze hängt auch vom gewählten Walzendurchmesser ab. Die Drehgeschwindigkeit kann beispielsweise zwischen 40 und 120 U/min liegen. Gut bewährt haben sich bei Walzendurchmessern von beispielsweise 18 bis 25 cm Drehgeschwindigkeiten zwischen 60 bis 80 U/min. Bei vertikaler Anordnung der Walze vor der Meßöffnung wird die Rotationsrichtung bevorzugt so ausgewählt, daß der Lack unter Ausnutzung der Schwerkraft auf die Walze gelangen kann.

Erst wenn die Küvette in entsprechender Weise an die rotierende Walze herangeführt wurde, kann das Lackmaterial in die Küvette eingefüllt werden. Durch die spaltförmige Öffnung im Boden der Küvette fließt das Lackmaterial augenblicklich auf die rotierende Walze und wird durch die Drehbewegung auf der Walzenoberfläche verteilt. Es bildet sich ein geschlossener Film über den gesamten Umfang der Walze aus, der in seiner Breite der Länge der spaltförmigen Öffnung entspricht.

Die Walze verbleibt vorerst solange in Drehbewegung, bis sich ein gleichmäßiger blasenfreier Film auf der Walzenoberfläche ausgebildet hat. Als vorteihaft hat es sich erwiesen, die Walze z.B. 0,5 bis 3 Minuten in Drehbewegung zu belassen. Die Schichtdicke des auf die rotierende Walze aufgebrachten Naßlackfilmes wird über die Breite der spaltförmigen Öffnung in der Küvette bzw. durch den Abstand zwischen Küvette und Walze eingestellt. Es können beispielsweise Schichtdicken von 0,2 bis 4,0 mm eingestellt werden. Als günstig haben sich beispielsweise Schichtdicken von 0,3 bis 2,0 mm, bevorzugt von 0,5 bis 1,0 mm erwiesen.

Sobald sich ein gleichmäßiger blasenfreier Film auf der Walzenoberfläche ausgebildet hat, wird, gegebenenfalls nach einer kurzen Ruhephase, die Walze oder bevorzugt die gesamte Einheit aus Küvette und Walze mit dem auf der Walze ausgebildeten Naßlackfilm, beispielsweise mittels einer beweglich gelagerten geeigneten Vorrichtung, an die Meßöffnung des Meßgerätes, insbesondere des Farbmeßgerätes herangeführt und die Messung durchgeführt; selbstverständlich kann auch das Meßgerät an die Walze herangeführt werden. Der Abstand zur Meßöffnung des Meßgerätes kann vorzugsweise durch geeignete Anschlagelemente eingestellt werden. Es hat sich als günstig erwiesen, den Abstand so einzustellen, daß er etwa 1 bis 4 mm größer ist als die Spaltbreite bzw. die Schichtdicke des Naßlackfilms. Die optische Messung kann bei stillstehender oder in Bewegung befindlicher Walze durchgeführt werden. Vorzugsweise wird die Messung an der rotierenden Walze vorgenommen, um unnötige Verunreinungen durch abtropfenden Lack zu vermeiden.

Als Meßgeräte können übliche Meßgeräte zur Bestimmung optischer Parameter, wie sie im Handel erhältlich und dem Fachmann bekannt sind, eingesetzt werden. Als Farbmeßgeräte können übliche zur Bestimmung von Remissionskurven geeignete Meßgeräte eingesetzt werden. Beispiele für einsetzbare Farbmeßgeräte sind das SF 600+ (Fa. Datacolor International) und das X-Rite MA 58 (Fa. X-Rite). Als auswertbare, farbmetrische Parameter werden z.B. Farbstärke und/oder Farbabstand erhalten. Die Auswertung kann rechnergestützt erfolgen. Die ermittelten Parameter werden mit den entsprechenden Werten einer Vorlage, z.B. eines Freigabemusters, verglichen. Dazu werden die entsprechenden Parameter der Vorlage in analoger Weise wie die der zu messenden Lackprobe gemessen und verglichen, wobei ebenfalls rechnergestützt ausgewertet werden kann. Auf Einzelheiten farbmetrischer Messungen muß hier nicht näher eingegangen werden. Sie sind dem Fachmann bekannt. Grundlagen und Prinzipien der Farbmessung sowie dazu geeigneter Farbmeßgeräte sind beispielsweise beschrieben in Römpp Lexikon Lacke und Druckfarben, Georg Thieme Verlag 1998, S. 220, 221, 223 und in Hans G. Völz, Industrielle Farbprüfung, VCH Verlagsgesellschaft mbH 1990.

Nach der Messung wird die Walze oder im bevorzugten Fall die Einheit aus Walze und Küvette vorteihafterweise wieder vom Meßgerät oder umgekehrt entfernt und es kann eine Reinigung erfolgen. Vorteilhaft ist es, die Walze auch während des Reinigungsvorganges weiterhin in Rotation zu belassen, um Verunreinigungen durch abtropfenden Lack zu vermeiden. Die Reinigung der Walze kann beispielsweise durch Abstreifen des Lackfilmes mit einer mit einer Abstreifkante versehenen Absaugeinrichtung in Kombination mit Lösemitteln erfolgen. Die Reinigung der Küvette kann beispielsweise durch Aussaugen des Lackmaterials mit einer Vakuumpumpe und weitere Reinigung mittels Ultraschall erfolgen. Um während des gesamten Prozesses eventuell auftretende Lackspritzer oder Lacktropfen aufzufangen, ist es vorteilhaft eine unter Walze und Küvette angeordnete Abtropfeinrichtung, z.B. eine Abtropfwanne, zu verwenden.

Nachstehend wird auch eine Vorrichtung zur Durchführung des erfindungsgemäßen Verfahrens beschrieben, die als Meßkopf für entsprechende Meßgeräte, insbesondere Farbmeßgeräte verwendet werden kann.

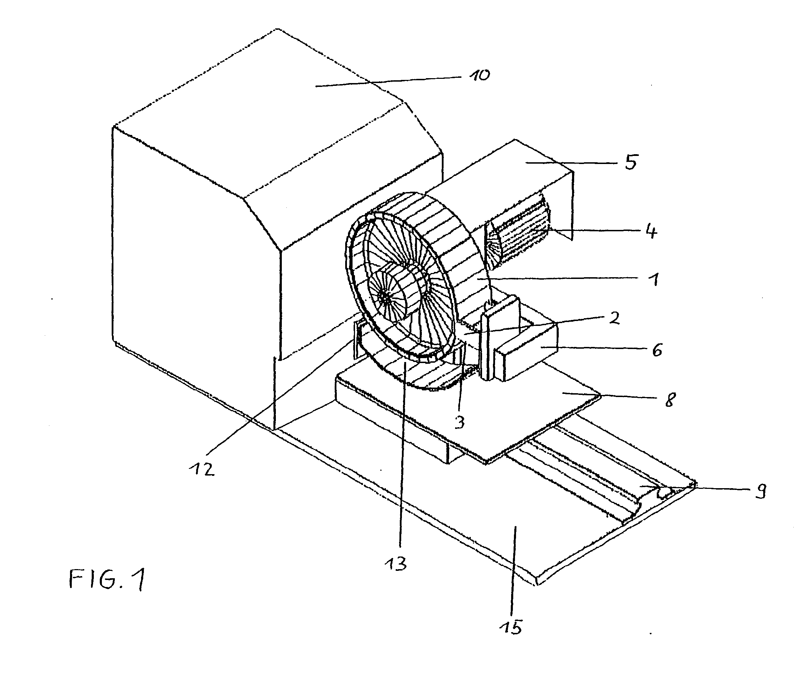

Figur 1 stellt eine Ausführungsform der Vorrichtung dar.

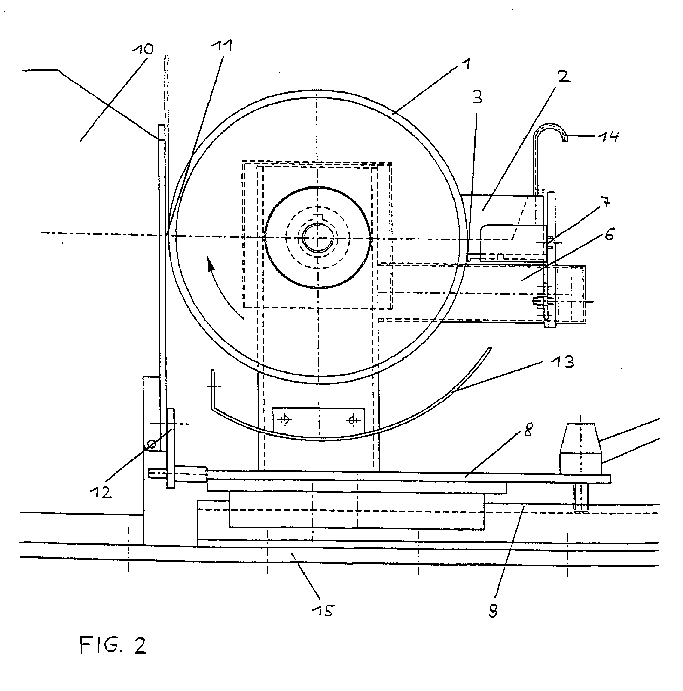

Figur 2 stellt eine Seitenansicht einer Ausführungsform der Vorrichtung dar.

In den Figuren stellen 1 eine antreibbare Walze aus einem ausgesparten Vollkörperzylinder mit Zylinderkern, 2 eine Küvette zur Aufnahme des zu messenden Lackes mit einer spaltförmigen Öffnung 3, über die der Lack auf die antreibbare Walze 1 gelangen kann und 4 eine Motor/Getriebe-Einheit mit einem Gehäuse 5 dar. Die Küvette 2 wird in einer Halterung 6 durch Federelemente 7 an der Walze 1 positioniert. Die Halterung 6 ist mit einer Trägerplatte 8 verbunden. Walze 1, Küvette 2, Halterung 6 und Motor/Getriebe-Einheit 4 sind auf der Trägerplatte 8 angeordnet. Die Trägerplatte 8 kann über Schienenelemente 9 bewegt und vor dem Meßgerät 10 mit der Meßöffnung 11 positioniert werden. Die Schienenelemente 9 sind über die lösbare Verbindung 12 mit dem Meßgerät 10 verbunden. Unter der Walze 1 ist auf der Trägerplatte 8 eine Abtropfwanne 13 zum Auffangen abtropfenden Lackes angeordnet. Zur besseren Handhabung weist die Küvette 2 einen Griff 14 auf. Das Farbmeßgerät 10, der gesamte Meßkopf auf der Trägerplatte 8 und die Schienenelemente 9 sind auf einer Grundplatte 15 lösbar angeordnet.

Das erfindungsgemäße Verfahren zur Messung optischer Parameter an insbesondere Naßlacken unterliegt keinerlei Beschränkungen hinsichtlich der Art der Lacke. Es ist einsetzbar zur Messung optischer, insbesondere farbmetrischer Parameter an beliebigen Lacken. Bei den Lacken kann es sich beispielsweise um mit Absorptionspigmenten pigmentierte farbgebende Lacke, sogenannte Unilacke, um mit Metallpigmenten, z.B. Aluminiumpigmenten und gegebenenfalls weiteren Pigmenten pigmentierte Metalleffektlacke oder um mit Interferenzpigmenten oder beliebigen weiteren Effektpigmenten pigmentierte Effektlacke handeln. In den Lacken können beliebige Pigmente miteinander kombiniert vorliegen. Die Messungen können sowohl an lösemittelbasierenden als auch wasserbasierenden Lacken durchgeführt werden.
Die weitere Zusammensetzung der Lacke, z.B. bezüglich der Bindemittel, Additive und sonstiger Lackbestandteile, ist unkritisch. Die Lacke müssen lediglich so beschaffen sein, daß ein problemloses Aufbringen auf den zylinderförmigen Träger möglich ist und die Ausbildung eines homogenen Filmes gewährleistet ist.

Das erfindungsgemäße Verfahren zur Naßlackmessung ermöglicht es, unabhängig vom verwendeten Farbmeßgerät, insbesondere unabhängig von der Anordnung der Meßöffnung am Farbmeßgerät, farbmetrische Messungen an Naßlacken schnell und effektiv mit der erforderlichen Meßgenauigkeit durchzuführen.

Das erfindungsgemäße Verfahren kann in der Lackindustrie Anwendung finden sowohl bei der Qualitätssicherung in der Lackfertigung und Lackstandardisierung als auch in den verschiedenen Entwicklungsstufen der Lackentwicklung. Insbesondere findet es Anwendung zur Auswertung von Zwischenergebnissen bei Tönprozessen in der Lackfertigung und Lackstandardisierung, beispielsweise bei der Herstellung von standardisierten Mischlacken oder standardisierten Pigmentpasten. Hierbei werden jeweils Zwischenwerte bestimmt, die noch relativ weit vom Endergebnis entfernt liegen und daher rasch und effektiv, jedoch mit der erforderlichen Genauigkeit ermittelbar sein sollen.

Ebenso ist eine Anwendung des erfindungsgemäßen Verfahrens beispielsweise auch im Bereich der Druckfarben möglich. Das erfindungsgemäße Verfahren sowie die Vorrichtung zur Durchführung des Verfahrens sind natürlich prinzipiell auch in beliebigen anderen Anwendungsbereichen anwendbar, wo generell optische und insbesondere farbmetrische Messungen an farbigen, flüssigen Medien erforderlich sind.

## Patentansprüche

1. Verfahren zur Messung optischer Parameter an flüssigen, farbigen Medien, **dadurch gekennzeichnet, dass** das flüssige, farbige Medium mittels einer Küvette auf einen sich in kontinuierlicher Bewegung befindlichen, walzenförmigen Träger aufgegossen, ein Film aus dem flüssigen, farbigen Medium auf dem sich in kontinuierlicher Bewegung befindlichen walzenförmigen Träger ausgebildet und an dem Film aus dem flüssigen, farbigen Medium optische Parameter unter Verwendung üblicher optischer Messeinrichtungen gemessen werden, wobei der Auftrag des flüssigen, farbigen Mediums auf den walzenförmigen Träger durch eine spaltförmige Öffnung, die in einer an die Walzenkrümmung angepassten Begrenzungsfläche der Küvette vorgesehen ist oder durch einen Spalt der zum walzenförmigen Träger offenen Küvette, der zwischen der Bodenfläche der Küvette und dem walzenförmigen Träger ausgebildet ist, aufgetragen wird.

2. Verfahren nach Anspruch 1, **dadurch gekennzeichnet, dass** als zu messende optische Parameter Helligkeit und/oder farbmetrische Parameter gemessen werden.

3. Verfahren nach Anspruch 2, **dadurch gekennzeichnet, dass** als farbmetrische Parameter Farbstärke und/oder Farbabstand gemessen werden.

4. Verfahren nach einem der vorhergehenden Ansprüche, **dadurch gekennzeichnet, dass** als flüssige, farbige Medien flüssige Lacke gemessen werden.

5. Verfahren nach einem der vorhergehenden Ansprüche, **dadurch gekennzeichnet, dass** die Messung an dem bewegten oder stehenden walzenförmigen Träger durchgeführt wird.

6. Verfahren nach einem der vorhergehenden Ansprüche, **dadurch gekennzeichnet, dass** der walzenförmige Träger horizontal oder vertikal vor dem Messgerät angeordnet wird.

7. Verfahren nach einem der vorhergehenden Ansprüche, **dadurch gekennzeichnet, dass** der walzenförmige Träger nach Ausbildung eines gleichmäßigen, blasenfreien Films auf seiner Oberfläche an die Messöffnung des Messgerätes herangeführt wird.

## Claims

1. A method for measuring optical parameters in respect of liquid coloured media, **characterised in that** the liquid coloured medium is poured by means of a cuvette onto a roller-shaped support which is in continuous motion, a film consisting of the liquid coloured medium is formed on the roller-shaped support which is in continuous motion, and optical parameters are measured in respect of the film consisting of the liquid coloured medium by using conventional optical measuring devices, the coat of the liquid coloured medium being applied onto the roller-shaped support through a slit-shaped aperture which is provided in a boundary surface of the cuvette that is adapted to the curvature of the roller or through a slit in the cuvette which is open towards the roller-shaped support, said slit being formed between the bottom surface of the cuvette and the roller-shaped support.

2. Method according to Claim 1, **characterised in that** brightness and/or colorimetric parameters are measured by way of optical parameters.

3. Method according to Claim 2, **characterised in that** colour intensity and/or colour difference are measured by way of colorimetric parameters.

4. Method according to one of the preceding claims, **characterised in that** liquid lacquers are measured by way of liquid coloured media.

5. Method according to one of the preceding claims, **characterised in that** the measurement is carried out on the moving or stationary roller-shaped support.

6. Method according to one of the preceding claims, **characterised in that** the roller-shaped support is arranged horizontally or vertically in front of the measuring instrument.

7. Method according to one of the preceding claims, **characterised in that** after formation of a uniform, bubble-free film on its surface the roller-shaped support is brought up to the measuring aperture of the measuring instrument.

## Revendications

1. Procédé de mesure des paramètres optiques sur des supports de couleur liquides **caractérisé en ce que** le support de couleur liquide est placé, via une cuve, sur un support cylindrique étant en mouvement continu, **en ce qu'**un film du support de couleur liquide et placé sur le support cylindrique étant en mouvement continu et **en ce que**, au niveau du film du support de couleur liquide, des paramètres optiques sont mesurés en utilisant les dispositifs de mesure optiques classiques, la couche du support de couleur liquide étant posée sur le support cylindrique via une ouverture en forme de fente percée dans une surface limite de la cuve, adaptée à la courbure de cylindre, ou via une fente de la cuve ouverte du support cylindrique, percée entre la surface du sol de la cuve et le support cylindrique.

2. Procédé selon la revendication 1 **caractérisé en ce que** la clarté et/ou les paramètres colorimétriques sont mesurés comme des paramètres optiques à mesurer.

3. Procédé selon la revendication 2 **caractérisé en ce que** l'intensité des teintes et/ou la différence chromatique sont mesurées comme des paramètres colorimétriques.

4. Procédé selon l'une quelconque des revendications précédentes, **caractérisé en ce que** les laques liquides sont mesurées comme des supports de couleur liquides.

5. Procédé selon l'une quelconque des revendications précédentes, **caractérisé en ce que** la mesure est effectuée sur le support cylindrique en mouvement ou fixe.

6. Procédé selon l'une quelconque des revendications précédentes, **caractérisé en ce que** le support cylindrique est placé à l'horizontale ou à la verticale avant l'appareil de mesure.

7. Procédé selon l'une quelconque des revendications précédentes, **caractérisé en ce que** le support cylindrique est amené, suivant la configuration d'un film uniforme sans bulle d'air, sur sa surface supérieure au niveau de l'ouverture de mesure du dispositif de mesure.
